(19)

Europäisches Patentamt
European Patent Office
Office européen des brevets

(11) **EP 3 702 781 A1**

(12) **EUROPEAN PATENT APPLICATION**

(43) Date of publication:
**02.09.2020 Bulletin 2020/36**

(51) Int Cl.:
**G01N 33/493** (2006.01)    **G01N 27/07** (2006.01)
**A61B 5/20** (2006.01)

(21) Application number: **19382142.8**

(22) Date of filing: **27.02.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Universitat Rovira I Virgili**
**43003 Tarragona (ES)**

(72) Inventors:
• **Andrade Mermet, Francisco Javier**
**43007 Tarragona (ES)**

• **Blanking, Pär Robert Erik William**
**43007 Tarragona (ES)**
• **Hoekstra, Rafael Wytze Fenwick**
**43007 Tarragona (ES)**
• **Ferré Albiol, Jordi**
**43007 Tarragona (ES)**

(74) Representative: **Hoffmann Eitle**
**Hoffmann Eitle S.L.U.**
**Paseo de la Castellana 140, 3a planta**
**Edificio LIMA**
**28046 Madrid (ES)**

(54) **INSTALLABLE SYSTEM FOR REGULAR CONTINUAL ANALYSIS OF AN INDIVIDUAL'S URINE TO DETERMINE THE LEVEL OF HYDRATION OF SAID INDIVIDUAL**

(57)    It is an object of the present invention to provide an indication of the state of health of an individual by monitoring changes in one or more characteristic constituents or properties of urine. In particular, the present invention provides an indication of the state of hydration of an individual with, preferably, the aim of determining any type of hydration imbalance. In order to achieve such indication of the state of health of an individual, the present invention provides a device or system capable of measuring conductivity and using such conductivity to estimate the volume of each micturition event.

FIG. 5

**Description**

**FIELD OF THE INVENTION**

The present invention relates to a system and method for monitoring the health of an individual by regular continual urine analyses. In particular, the present invention relates to a system and method for monitoring the hydration level of an individual by regular continual urine analyses.

**BACKGROUND OF THE INVENTION**

Water is the primary ingredient of life. Despite this, our means to measure its levels within the human body are limited and a gold standard method of hydration assessment remains elusive. While the dictionary definition of euhydration- or being in water balance- is easy, establishing the physiological definition is not. Nevertheless, hydration is critical and as the current medical paradigm thirsts for objective measurements, there are numerous efforts underway to develop tools and quantify hydration.

Imbalances of water and electrolyte levels are involved with numerous disorders- either as a cause or as a result. These include hypernatremia, hyponatremia, polyuria, polydipsia, hypokalemia, hyperkalemia, sickle cell anaemia, diabetes insipidus, and chronic renal failure, and hypothyroidism. The maintenance of minerals and water is regulated by the kidneys and is affected by three factors: circadian rhythms, osmoregulation, and volume regulation. Hydration assessment techniques can be divided into subjective and objective methods. Subjective observations include skin turgor, thirst and mucous membrane moisture. These methods are not deemed reliable, so objective methods remained attractive. Among such objective methods, uroanalytic methods offer some advantages as they are non-invasive, and tend to be low-cost with the possibility of using portable meters. The established methods for hydration assessment in urine are osmolarity, specific gravity, conductivity, colour and 24-hour volume. These have been found to have medium to strong positive correlations with each other, although, it has been stated that urinary measures may have a time lag over the short term. In this sense, it has been recommended that future research efforts be focussed on novel assessment techniques that measure fluid volume and concentration in real time with excellent precision, accuracy and reliability, while being portable, inexpensive, safe and simple, non-invasive, with results that can be interpreted in concert with other hydration indices. This is indeed a tall order, but one that the present invention aims to contribute towards.

With a growing world population and a straining medical system that remains unaffordable to many, new paradigms in healthcare are paramount. Low-cost distributed diagnostic tools are one aspect of this. Indeed, by moving the analyses out of the laboratory, simpler and more affordable tools can be used to arrive at meaningful diagnostic information with a modest compromise in analytical performance. These tools often fall within the categories of point-of-care devices, wearables and decentralized diagnostic instruments, for which the glucometer has been the flagship product. This burgeoning field of portable, low-cost and robust decentralized chemical diagnostics is now beginning to interface with the more established field of miniaturised, wireless and flexible electronics and the marriage of these two are promising access to a wealth of new chemical information. Furthermore, where traditionally an individual's physiology was examined against an average of the population, the tools are now appearing to track an individual over the long-term, allowing for baseline levels and patterns of the individual to be established. When connected to the internet, this information can be called the Internet-of-you and opens the door to a new realm of insightful personalized analytics.

**BRIEF DESCRIPTION OF THE DRAWINGS**

The present invention will be understood and appreciated more fully from the following detailed description taken in conjunction with the drawings in which:

**Figure 1.** Schematic representation of the Smart Toilet shown in section view. Left: full device complete with funnel and electronics housing. Right: detail of the funnel.

**Figure 2.** Plot of the conductivity readings ($\kappa$) of 19 urine samples validated against the readings of a commercial conductivity meter. The values obtained from both methods were compensated for the temperature at which they were taken, and are here provided on the same scale, at 25°C.

**Figure 3.** The Smart Toilet installed with tablet computer connected and mounted to the wall.

**Figure 4.** The relationship of volume to the time of signal. (Error bars represent 1 standard deviation; N = 3).

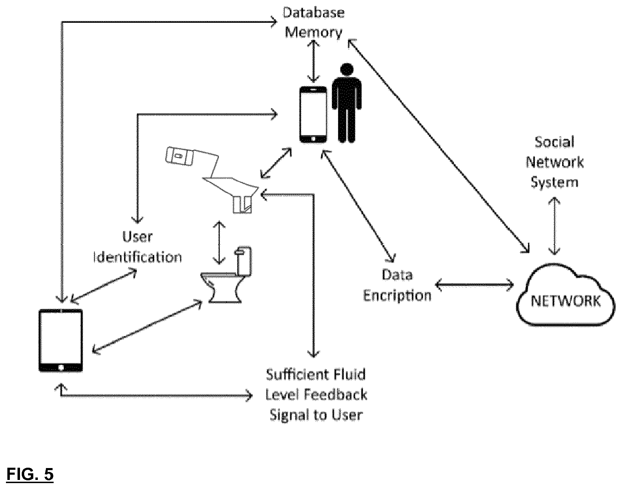

**Figure 5.** This figure represents the general concept of the present invention.

Similar elements in the Figures are numbered with similar reference numerals.

## DESCRIPTION OF THE INVENTION

The present invention provides a portable system and method for periodic collection of urine from one or more individuals, sampling the urine and analysing data obtained therefrom. The system analyses the data collected over a predefined time period, typically several weeks. The system is easily transportable and usable with standard conventional commercially available sanitary installations on which it is easily installable.

In particular, it is an object of the present invention to provide an indication of the state of health of an individual by monitoring changes in one or more characteristic constituents or properties of urine, in particular, the present invention provides an indication of the state of hydration of an individual with, preferably, the aim of determining any type of hydration imbalance. In order to achieve such indication of the state of health of an individual, the present invention provides a device or system capable of measuring conductivity and using such conductivity to estimate the volume of each micturition event.

In particular, a first aspect of the present invention provides a system suitable for measuring the hydration state of an individual, said system comprising a toilet unit mountable on a toilet bowl or to any other sanitary installation, said portable toilet unit further comprising: a collection and sampling sub-unit for collecting and measuring the electrical conductance of urine; wherein said sampling sub-unit comprises one or more means for measuring conductivity; wherein at least one of the means for measuring conductivity is a non-faradaic conductimetric system comprising a pair of electrodes to which an alternate current is applied, preferably between 10 and 100 Hz, more preferably between 50 and 60 Hz, still more preferably about 60 Hz, to revert the polarity of the electrodes in each half cycle (it is noted that alternatively, pulsed DC supply can be also applied with the same purpose); and wherein the system further comprises an electronics sub-unit which uses the electrical conductance measurement of the urine obtained from the non-faradaic conductimetric system, from which, by previous calibration, the electronics sub-unit provides the ionic concentration and the volume of each micturition; wherein optionally said system further comprises one or more means for measuring temperature.

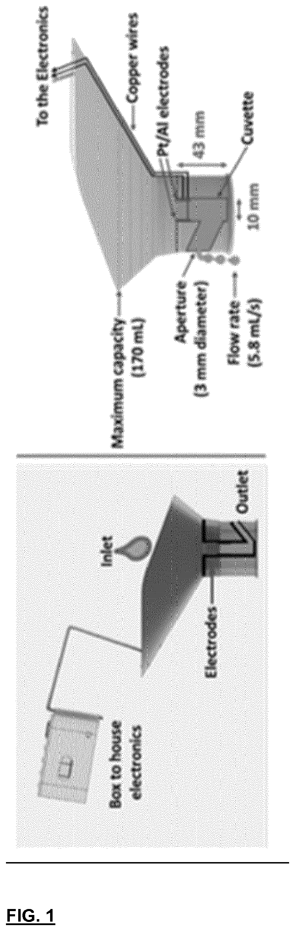

A schematic illustration of such system is depicted in figure 1.

It is noted that in the system of the first aspect of the invention, the volume of each micturition event is conductometrically determined by the electronics sub-unit by identifying a disruption in the conductivity of the system, when water or other solution in the sampling sub-unit is replaced by urine, wherein when said disruption takes place such event marks or triggers the initial time of the micturition event. When, on the other hand, such conductivity is stabilized, such stabilization event indicates that the micturition event has ended and thus marks the final time or termination of said event. The time interval between the initial time and the final time is then used to calculate the volume of each micturition event as reflected below.

In this sense, theoretically, the speed of efflux, v, of a fluid from a hole at the lower part of a container is predicted by Torricelli's Law (Equation 8.1), which states that v is proportional to the acceleration due to gravity (g) and the height (h) of the fluid.

$$v = \sqrt{2gh} \quad \text{(Equation 8.1)}$$

One outcome of this equation is that as the height of the fluid column changes during flow, so too does the flow rate. From Torricelli's Law, an equation for the time required to empty a cylindrical container may be derived as follows. For a cylinder of cross-sectional area, A, and an opening of cross-sectional area a, filled with liquid to a height h, the velocity of efflux v, may be described as:

$$v = \frac{dx}{dt} = \sqrt{2gh}$$

$$dx = \frac{A\,dh}{a}$$

$$\frac{dx}{dt} = \frac{A\,dh}{a\,dt} = \sqrt{2gh}$$

$$\frac{A\,dh}{a\sqrt{2gh}} = dt$$

$$\frac{A}{a\sqrt{2g}}\int_0^h \frac{dh}{\sqrt{h}} = \int_0^t dt \qquad \text{Which integrates to:}$$

$$t = \frac{2A}{a\sqrt{2g}}\left(\sqrt{h_i} - \sqrt{h_f}\right) \qquad \text{Where } h_i \text{ and } h_f \text{ respectively represent the initial and final height of the liquid}$$

The change in time during efflux, $\Delta t$ may then be written as:

$$\Delta t = \frac{2A}{a\sqrt{2g}}\sqrt{\Delta h} \qquad \text{And because } \Delta h = \frac{\Delta V}{h}$$

$$\Delta t = \frac{2A}{a\sqrt{2g}}\sqrt{\Delta V / A} \qquad \text{(Equation 8.2)}$$

In this way, a relationship between the volume of liquid and the time of efflux can be established for a cylinder. It is noted that during prototyping, a cylindrical device was tested, and the power law relationship described by Equation 8.2 was indeed found to operate in practice. However, in order to tailor a device towards application within a toilet, a cylindrical vessel is not optimal. Rather, a more bowl-shape is preferable. Indeed, such a shape is also advantageous in calculations. This is because while its volume-to-time relationship would be far more complex to theoretically derive, in practice this shape drives the relationship of volume to time towards linearity. This is because, while within a cylinder, the pressure decreases dramatically as the vessel empties, a bowl-shaped device maintains a more constant pressure during efflux. In fact, a shape with a uniform rate of efflux can be mathematically described [Wikipedia.org, https://en.wikipedia.org/wiki/Torricelli's_law, (accessed 4 June 2018)].

In any case, an experimental approach is recommendable as each particular design will differ in its modulation of flow. The practical considerations involved in a Smart Toilet-specifically the need to limit the size of reservoir that must be prevented from overflowing- make rapid flow most suitable, which was achieved by optimizing the outlet area. This also tended to increase the linearity of the volume to flow time relationship, which was optimized at a flow rate of approximately 5.8 mL/s. In the case of the device presented here, this relationship slightly diverged from linearity at volumes below 100 mL. Volumes below 30 mL flowed too rapidly through the device to be detected reliably. Accordingly, in a preferred embodiment of the invention, volume can be calculated in the following manner as written into a computer compatible code: if the signal time corresponded to a volume below 30 mL, "insufficient volume" was printed; if the signal time corresponded to a volume between 30 and 100 mL, a suitable equation can be used; if the signal time corresponded to a volume greater than 100 mL, a further suitable equation can be used. The triplicate calibration can be easily made by pouring 100 mM NaCl through the Smart Toilet in a range of volumes. Following the protocol of calculations described above, the recovery for each volume across this range can be in range of 99.9 $\pm$ 2.5 %.

Given that mean urination rates are reported as 13.05 $\pm$ 6.12 mL/s and mean maximum rates as 22.5 $\pm$ 9.2 mL/s, future prototypes could be easily calibrated for allowing these faster flow rates.

The system of the present invention thus comprised of a "toilet unit" which further comprises an electronics sub-unit such as a local main processing unit (MPU) and/or an electronic and communication system.

Without being limiting, the MPU may be a microprocessor or microcontroller. A separate unit, generally, but not necessarily, mounted on a wall, herein designated as a "wall unit", includes inter alia a user interface and a local MPU which stores and handles data transferred to a database. The MPU in the wall unit also may be a microprocessor or microcontroller. The toilet and wall units exchange information by a communications link.

Without being limiting, the electronic and communication system of the present invention might include the following general elements: a central processing unit (CPU); a database memory, a means for inputting information, a user recognition system, a power supply and, at least, one communication hub that collects and forwards the data to a data recording and a suitable display. In the present invention, ideally the electronic and communication hub is mounted within the device. Preferably, the communication hub is perfectly isolated from the entrance of any type of fluid, humidity and/or may be waterproof. The communication hub may be custom-built based on commonly available wireless chip sets for different communication protocols and standards such as (but not limited to) Wi-Fi, NFC, and RFID Bluetooth, Bluetooth low-energy (BLE) or ANT+. The hub may further comprise any type of suitable batteries or power supply to power the system. Additionally, the batteries can be disposable after a given use, rechargeable or the unit can be self-powered by any suitable mechanism. In another case where may be important to monitor urine in large scale surveys, different units may be simultaneously transmitting to a central hub located within an optimal coverage range.

It is noted that the CPU or the MPU can detect, as explained above, when the volume to flow time relationship diverges from linearity, such as for example at volumes below 100 mL. Accordingly, in a preferred embodiment of the invention, volume can be calculated by the electronics subunit by duplicate or triplicate calibrations or more so as to adapt the system to different urine volume as well as to different urination rates.

In a preferred embodiment of the first aspect of the invention, the collection and sampling sub-unit comprises a sampling syphon system capable of maintaining, by a change of fluid pressure between the sampling sub-unit and the highest orifice of the siphon, the minimal constant volume of urine needed in the sampling sub-unit to perform the conductivity analytical measurements. However, measurements can be also performed under a continuous flow approach. In this sense, for obtaining an optimal measurement of the conductivity and estimate the volume of each micturition event, the collection and sampling sub-unit comprises a sampling syphon system that has a shape with a uniform rate of efflux and allows stagnating the sample in the solution holder through a fully automated sampling-siphon system. Precisely, the fluid level in the sampling-siphon system is auto-regulated by a change of fluid pressure between the solution holder and the highest orifice of the siphon leaving a minimal constant volume of sample in the cuvette to perform the conductivity analytical measurements. Additionally, the system can work with any given urine sample added with sufficient volume to fully eliminate the previous one, without necessarily needing calibrations through flushing with water in between sample introductions. Ideally, in the present invention, the water used while flushing the toilet is used to clean the sample and calibrate the system periodically. The siphon that can be used in the present invention is ideally (but not limited to) any tube made of any material facilitating the fluids discharge. Furthermore, the sampling-siphon system allows the use of the system without user-intervention by:

1) Auto-cleaning the sample.
2) Auto-calibrating the system.

In other cases where toilets may be waterless, alternative systems to clean the sample and re-calibrate the system can be used, should cleaning and calibration be required. For example, a suitable cartridge with water-based solution can be easily integrated to flush water after each use. Overall the parts in the device in contact with urine are preferably made of a material or various materials that are anti-bacterial and/or odour-free in a way that the device is kept hygienic even if it is not flushed with water between sample collections. This is particularly important in situations when flushing is not possible such as in waterless and dry toilets, urinals and/or other sanitary installation, or when a previous sample collection have not been followed by a water flushing.

In another embodiment of the first aspect of the invention, the system further comprises a urine funnel capable of transferring the urine to the collection and sampling sub-unit.

In another embodiment, the system comprises means to avoid the entrance of air bubbles into the urine solution present in the collection and sampling sub-unit.

The analysed concentrations of urine constituents that make up for the total ionic concentration measured by the present system, are, for example, electrolytes such as sodium, potassium, chloride carbonate, whose changes provide indications of a person's health or body condition, such ionic concentration is preferably recorded by the system of the first aspect of the invention. It is readily understood that the aforementioned constituents are exemplary only and they are not intended to be limiting.

A personal database of the urine properties and constituents measured can be established for each individual using the toilet or other sanitary installation. Data is analysed on the basis of the individual's last readings, generally a series of readings. An exception is indicated when there is a substantial deviation of the most recent measurement of an analysed constituent or property from its average value, typically the average obtained over a predefined period. The average may be a moving average. While generally an average is used, other statistical measures, such as the standard deviation, may be used instead. Therefore, when average is discussed herein it is to be considered as exemplary only and not to be considered limiting.

In the present invention, a user recognition system can be used to identify the user whose urine is collected. Systems such as (but not limited to) digital code, facial, voice, fingerprint, iris, retinal scan and/or NFC can be implemented.

Additionally, a system to provide feedback regarding the valid collection of urine to perform the analytical measurement is integrated. Commercially available systems for providing feedback could be any type of (but not limited to) sound, light or vibration sensor. Furthermore, a system comprising any type of (but not limited to) light and/or movement sensor can also be used to turn on and off the detection system with the aim to increase the efficiency of the power supply and durability of the batteries, especially when the system is in stand-by mode.

According to a further aspect of the present invention, the system of the first aspect of the invention or of any of its preferred embodiments, is deemed a medical analysis system. The system includes a toilet unit mountable on a toilet bowl so as to preferably be readily attachable and detachable therefrom. The toilet unit includes a collection and sampling sub-unit for collecting and measuring the characteristics of urine as described in the first aspect; an electronics sub-unit for data collection and processing of the measured characteristics; a first communications link for facilitating data transfer between the collection and sampling sub-unit and the electronics sub-unit; and means for removably fastening the collection and sampling sub-unit inside a toilet bowl, operative to permit ready positioning of the sub-unit in and removal from any of a plurality of toilet bowls as desired. The system also includes a wall unit having a second communications link for facilitating data transfer between the wall and toilet units. The wall unit further includes means for storing and further processing the data to determine if changes have occurred in the one or more measured characteristics of urine.

In another embodiment of the system of the present invention, the system further includes output means operative in response to a change in the measured one or more characteristics of urine, the change being greater than a predetermined statistical threshold value, thereby to provide an output indication indicating that the individual requires further medical examination. The output means is chosen from a group consisting of a display displaying a text message; a display displaying a numerical value; an audio alarm; an alarm lamp; and a central institutional computer providing a text message.

In yet another embodiment of the system of the present invention, the means for removably fastening is a flexible strap, the strap in attachment with the collection and sampling and electronics sub-units, and positionable over a lip of the toilet bowl. In other embodiments the means for removably fastening is a rigid element, shaped to be positionable on a lip of a toilet, the rigid element in attachment with the collection and sampling sub-unit and the electronics sub-unit. In yet other embodiments the means for removably fastening is a hanger-like element, the element in attachment with the collection and sampling sub-unit and the electronics sub-unit, and shaped to be positionable on a lip of a toilet. Ideally the system is installed within the toilet bowel or any other sanitary installation including (but not limited to) urinal or bidet through any suitable fixation system such as (but not limited to) glues, suction caps, straps and/or hooks. Alternatively, the system can be detachable, portable and usable with different decentralized cases and/or sanitary installations. The system can be used by any type of gender and age group.

A further aspect of the invention refers to a system, as specified in any of the embodiments above, for determining the level of hydration of an individual. Furthermore, the assessment of hydration status can be performed multiple times among customized periods of time such as (but not limited to) hours, days, weeks, months and/or years providing acute and long-term hydration insights. Additionally of hydration status, the system of the present invention can provide indications regarding the fluid intake of a person.

Another aspect of the present invention refers to a "non-therapeutic" method for assessing and discriminating the level of hydration.

Preferably the system may be used multiple times. The system according to the present invention may be used indefinitely. The system can be used by any type of gender and age group.

The system in the present invention can be used in different fields. In this sense, sports, fitness, well-being, healthcare, medical, food and beverages, and the military sector are potential fields to monitor hydration levels. Opportunities for the groups at higher risk of dehydration such as children, elderly, pregnant women, breastfeeding women, athletes or outdoor workers may be present. Also, people that are undergoing some health issues, such as diarrhoea or fever, may be prone to dehydration and thus prone to use the system to monitor hydration status. In this sense, for children and elderly this system could help to educate of the importance of being hydrated. Cognitive impairment, bad mood, obesity, lower academic outcomes, hospitalizations or even mortality are some of the consequences of dehydration for these groups. For pregnant women hydration is crucial to support the growth of the fetus since water regulation dynamics are increased. In the case of breastfeeding women, hydration becomes particularly important since the production of breast milk significantly increases a mother's water loss. For athletes the system of the present invention could be used as a suitable to monitor hydration and fluid intake before and after trainings and/or competitions in order to avoid the_decrease of sports' performance and health related disorders such as heat stress or injuries. For health organizations and key industry players to have access to large sets of data on hydration status could represent a change in the current knowledge gap and facilitate supporting initiatives for improving the hydration of the population or personalized drinks.

In all cases, the system can be used to monitor hydration status and propose corrective actions if required.

Other field of interest are the cosmetic and beauty where this technology could be applied regarding skin health.

In this sense, being hydrated play an important role in different skin functions, such as lubricant, shock absorbent, the water "barrier" function or the "envelope" function, and water deficiency is associated with several dermatological dysfunctions.

Other field of interest are the sanitary or smart homes where this technology could help to become everyday objects such as toilets, urinals and/or bidets into smart objects through embedded systems capable to monitor hydration status.

A further aspect of the invention refers to a method for measuring or determining the hydration level of an individual, wherein said method includes the steps of: positioning the system, as specified in any of the embodiments above, for collecting and measuring characteristics of urine in a toilet bowl or in a sanitary device; collecting urine passed by an individual within the means for collecting and measuring; measuring the value of the characteristics of the collected urine; comparing the measured value of the characteristics of the collected urine with a pre-determined statistical threshold for that characteristic, the statistical threshold being calculated from a pre-determined series of similar measurements of that characteristic; and indicating that the measured value exceeds the statistical threshold of the at least one measured characteristic of urine, thereby to indicate changes in an individual's urine which may signal the individual's hydration levels. Preferably, the method includes the use of the system as defined in any of the previous embodiments for measuring or determining the hydration level of an individual.

Additionally, the system can be designed as a standalone, fully integrated portable unit that can be used in field tests without being connected to a toilet or any other device.

Finally, the present invention includes the step of comparing the measured value of the optical property of urine associated to urine colour for assessing hydration levels to different custom-based hydration thresholds. These thresholds are obtained based on a wide range of predetermined analytical measurements that have been statistically treated regarding their validity, sensitivity, specificity and level of significance. Additionally, other statistical approaches can be selected. The thresholds are discriminated through a quantitative value that is related to a nominal scale of hydration status. Alternatively, the values can also be expressed by another mathematical and/or statistical means such as (but not limited to) percentage, nominal ranges and/or differences. The nominal scale to compare our data that is used in the present invention is known in the art and refers to, very well hydrated, hydrated, dehydrated and severely dehydrated.

It will be appreciated by persons skilled in the art that the present invention is not limited by what has been particularly shown and described in the examples below.

## Examples

### - Reagents and materials

Commercial standard solutions from Mettler-Toledo (traceable to NIST values) of 1.413 and 12.88 mS/cm (25 °C) KCl were used for calibration of the commercial conductivity meter and to determine the conductivities of solutions of 0.01 M and 0.5 M KCl solutions prepared in-house. Volume estimations were calibrated using a 100 mM NaCl solution. Aluminium foil was purchased from a supermarket.

### - Instrumentation and measurements

Analytical grade graduated cylinders were used to calibrate the Smart Toilet's volume estimation. An EZO conductivity circuit was used in conjunction with an Arduino Nano V 3.1. A 100 nm layer of platinum was sputtered upon aluminium foil. Reference conductivity measurements were made with a commercial conductivity meter at room temperature (approximately 19°C). The conductivity measurements of the Smart Toilet were made in situ, and were therefore assumed to be at the body temperature of 37 °C. Both instruments were calibrated for temperature accordingly. A Teclast Ultrapad (X80 Pro W32GB DG) tablet computer from Guangzhou Shangke Information Technology Limited was used for communication with the Arduino.

### - The 'SmartToilet' device

### - Device structure

The Smart Toilet device was 3D printed. The funnel area was oval in shape with a collection capacity of 170 mL. This collected urine into the cuvette of 1 cm$^2$, to fill its 4.3 cm height during micturition events. The outlet aperture size was optimized to be 3 mm in diameter to limit the flow rate.

The cuvette was housed inside using silicone glue to ensure a watertight seal. Inside and in the upper section of the cuvette, two square electrodes of platinum upon aluminium foil were placed, each 8 mm$^2$. A small hole was drilled through either side of the cuvette behind the electrodes through which a copper wire was threaded and held in place with conductive epoxy glue. In this way, the electrodes were separated by 1 cm on the horizontal plane, and remained disconnected until the cuvette was filled as they were situated above the waterline of the cuvette at equilibrium.

The Smart Toilet was designed to fit a standard Western toilet. It was located at the front of the toilet to collect urine from a sitting user- male or female- while not interfering with other operations of the toilet. The front-most edge of the device was oriented so as to collect water when the toilet was flushed and in doing so, rinse the device. Schematics of the device are shown in Figure 8.2 and it is photographed within a toilet in Figure 8.4. The Arduino microcontroller and EC EZO conductivity circuit are located inside a 3D printed plastic box outside of the toilet.

#### - Device electronics

The copper wires connected to the electrodes were used as inputs into the EZO conductivity circuit. This in turn, communicated with the Arduino Nano microcontroller using two digital pins for RX/TX communication in UART Mode. The Arduino was connected to a tablet computer via USB port to print data to the serial monitor, and this tablet was mounted on the wall beside the toilet.

#### - Arduino code

An Arduino sketch was written to continuously read the conductivity. It defined a micturition event as the duration of the conductivity exceeding 2 mS/cm (at 25 °C). This cut-off level ensured that flushing events were not recorded. The duration of each micturition event was recorded and volume was calculated based on an established relationship between time and volume specific to the fluidics of the device.

#### - Results and discussion

#### - Conductivity determinations

The EC EZO conductivity circuit is the size of a US quarter dollar and reads conductivity across the range of 0.07 - 500,000 μS/cm with an accuracy of ± 2 % with temperature compensation (H. Keller, Architecturaldigest.com, https://www.architecturaldigest.com/story/kohler-numi-smart-toilet-amazon-alexa-craziest-launch-at-ces, (accessed 19 June 2018). Within the Smart Toilet, it was connected to the platinum electrodes to read conductivity of urine passing through. Prior to initial use, it was calibrated dry, followed by a two-point calibration with 1.523 and 55.5 mS/cm (25°C) solutions of KCl. This was in accordance with the instructions of the datasheet. It was then set to read conductivity of solutions at 37°C- again, using input commands specified in the datasheet.

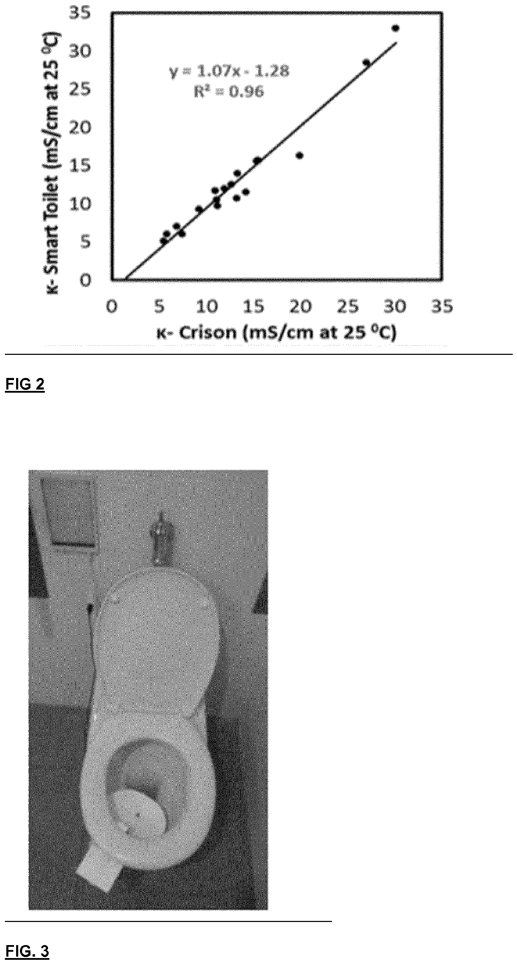

After being connected via USB cable to a wall-mounted tablet computer, people were asked to urinate through the Smart Toilet and to also collect a small volume for subsequent validation. The conductivity and volume of each micturition event were immediately printed out on the tablet. The system was set to disregard values below 2 mS/cm (at 25°C) to avoid false readings from flushing of the toilet. The 6 female and 13 male urine samples were analysed within this trial and the conductivity values were validated by analysing the collected portions with a commercial conductivity meter. This revealed a positive bias of 21 % which can be attributed to the measurements being made in a matrix distinct from the aqueous KCl solutions used for calibration. As this bias was consistent across all samples, a simple correction factor was added to the calculations. For both instruments, temperature differences were accounted for during calibration and the agreement between both readings was excellent (Figure 8.3). The conductivity values recovered by the Smart Toilet were 97.0 ± 9.5 % of those of the commercial instrument (see figure 2).

The Smart Toilet's medium term stability in conductivity determinations was evaluated by assessing twenty-one micturition events during the next month and validating in the same way as previously described. No subsequent calibration was made to the device during this time. The conductivity values remained accurate yielding recoveries of 104.3 ± 7.5 %.

#### - Volume estimations

Theoretically, the speed of efflux, v, of a fluid from a hole at the lower part of a container is predicted by Torricelli's Law (Equation 8.1), which states that v is proportional to the acceleration due to gravity (g) and the height (h) of the fluid.

$$v = \sqrt{2gh} \text{ (Equation 8.1)}$$

One outcome of this equation is that as the height of the fluid column changes during flow, so too does the flow rate. From Torricelli's Law, an equation for the time required to empty a cylindrical container may be derived as follows.

For a cylinder of cross-sectional area A, and an opening of cross-sectional area a, filled with liquid to a height h, the velocity of efflux v, may be described as:

$$v = \frac{dx}{dt} = \sqrt{2gh}$$

$$dx = \frac{A\,dh}{a}$$

$$\frac{dx}{dt} = \frac{A\,dh}{a\,dt} = \sqrt{2gh}$$

$$\frac{A\,dh}{a\sqrt{2gh}} = dt$$

$$\frac{A}{a\sqrt{2g}} \int_0^h \frac{dh}{\sqrt{h}} = \int_0^t dt$$   Which integrates to:

$$t = \frac{2A}{a\sqrt{2g}}\left(\sqrt{h_i} - \sqrt{h_f}\right)$$   Where $h_i$ and $h_f$ respectively represent the initial and final height of the liquid

The change in time during efflux, $\Delta t$ may then be written as:

$$\Delta t = \frac{2A}{a\sqrt{2g}}\sqrt{\Delta h}$$   And because $\Delta h = \frac{\Delta V}{h}$

$$\Delta t = \frac{2A}{a\sqrt{2g}}\sqrt{\Delta V / A}$$   (Equation 8.2)

In this way, a relationship between the volume of liquid and the time of efflux can be established for a cylinder. It is noted that during prototyping, a cylindrical device was tested, and the power law relationship described by Equation 8.2 was indeed found to operate in practice. However, in order to tailor a device towards application within a toilet, a cylindrical vessel is not optimal. Rather, a more bowl-shape is preferable. Indeed, such a shape is also advantageous in calculations. This is because while its volume-to-time relationship would be far more complex to theoretically derive, in practice this shape drives the relationship of volume to time towards linearity. This is because, while within a cylinder, the pressure decreases dramatically as the vessel empties, a bowl-shaped device maintains a more constant pressure during efflux. In fact, a shape with a uniform rate of efflux can be mathematically described [Wikipedia.org, https://en.wikipedia.org/wiki/Torricelli's_law, (accessed 4 June 2018)].

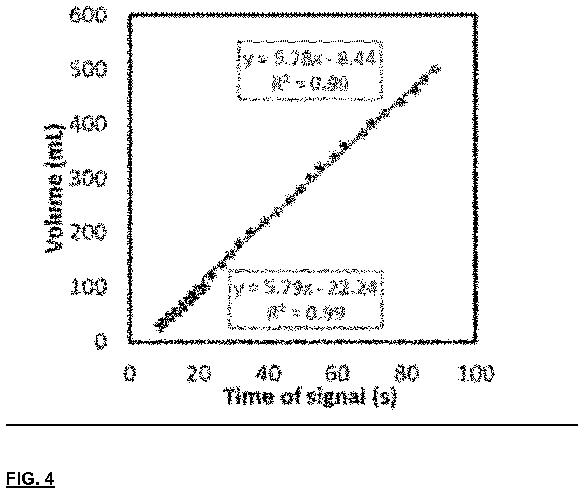

In any case, an experimental approach is recommendable as each particular design will differ in its modulation of flow. The practical considerations involved in a Smart Toilet-specifically the need to limit the size of reservoir that must be prevented from overflowing- make rapid flow most suitable, which was achieved by optimizing the outlet area. This also tended to increase the linearity of the volume to flow time relationship, which was optimised at a flow rate of approximately 5.8 mL/s. In the case of the device presented here, this relationship slightly diverged from linearity at volumes below 100 mL (Figure 4). Slightly different equations were therefore used- these are shown in Figure 4. Volumes below 30 mL flowed too rapidly through the device to be detected reliably. Accordingly, volume was calculated in the following manner as written into the Arduino code: if the signal time corresponded to a volume below 30 mL, "insufficient volume" was printed; if the signal time corresponded to a volume between 30 and 100 mL, one suitable equation was used; if the signal time corresponded to a volume greater than 100 mL, a further suitable equation was used. The triplicate calibration in Figure 4 was easily made by pouring 100 mM NaCl through the Smart Toilet in a range of volumes. Following the protocol of calculations described above, the recovery for each volume across this range was 99.9 $\pm$ 2.5 %.

Given that mean urination rates are reported as 13.05 $\pm$ 6.12 mL/s and mean maximum rates as 22.5 $\pm$ 9.2 mL/s [25], future prototypes can be easily made by allowing for these faster flows.

- **Conclusions**

A 'Smart Toilet' to measure conductivity and estimate volume of micturition events is herein described. This *system* was inspired by the ancient water clocks and used the inverse principle upon which they operated. Surprisingly and despite the simplicity of the device, this system was demonstrated to function autonomously to record the conductivities of 19 real samples *in situ* within a toilet with an accuracy of 97.0 ± 9.5 %. In fact, the algorithm to estimate volume performed with an accuracy of 99.9 ± 2.5 % across the range of 30-500 mL. The device was shown to continue performing without calibration for at least one month. The entire process of signal detection and interpretation was automated using a simple Arduino microcontroller which printed out the values of conductivity and volume on a wall-mounted tablet computer to provide real-time information to the user.

It is proposed that the data generated could contribute to the tracking of hydration levels for healthcare and optimization of athletic performance.

**Claims**

1. A system suitable for measuring the hydration state of an individual, said system comprising a toilet unit mountable on a toilet bowl or to any other sanitary installation, said portable toilet unit further comprising: a collection and sampling sub-unit for collecting and measuring the electrical conductance of urine; wherein said sampling sub-unit comprises one or more means for measuring conductivity; wherein at least one of the means for measuring conductivity is a non-faradaic conductimetric system comprising a pair of electrodes to which an alternate or pulsed direct current is applied to revert the polarity of the electrodes in each half cycle; and wherein the system further comprises an electronics sub-unit which uses the electrical conductance measurement of the urine obtained from the non-faradaic potentiometric system, from which, by previous calibration, the electronics sub-unit provides the ionic concentration and the volume of each micturition.

2. The system of claim 1, where the system further comprises a urine funnel capable of transferring the urine to the collection and sampling sub-unit.

3. The system of claim 1, where the system further comprises one or more means for measuring temperature.

4. The system of any of claims 1 to 3, wherein the collection and sampling sub-unit comprises a sampling syphon system capable of maintaining, by a change of fluid pressure between the sampling sub-unit and the highest orifice of the siphon, the minimal constant volume of urine needed in the sampling sub-unit to perform the conductivity measurements, wherein the collection and sampling sub-unit comprising the sampling syphon system has a shape with a uniform rate of efflux so that the urine's volume can be measured.

5. The system of any of claims 1 to 4, wherein the system comprises means to avoid the entrance of air bubbles into the urine solution present in the collection and sampling sub-unit.

6. The system of any of claims 1 to 5, wherein said electronics sub-unit and the toilet unit are integrated into a single unit.

7. The system according to claim 6 wherein said electronics sub-unit includes at least one of the following elements: a main processing unit; a database memory; a means for inputting information; a user recognition system; a communication hub that collects and forwards the data to a data recording and a suitable display; and an internal power supply.

8. *Ex vivo* use of the system of any of claims 1 to 7 for determining the level of hydration of an individual.

9. An *ex vivo* method for measuring the volume of one or more micturition events of an individual, wherein said method includes the steps of: positioning the system, as specified in any of the precedent claims, for collecting and measuring characteristics of urine in a toilet bowl or in a sanitary device; collecting urine passed by an individual within the means for collecting and measuring; measuring the value of the conductivity of the collected urine by the system; wherein the volume of each micturition event is conductometrically determined by the electronics sub-unit of the system by identifying a disruption in the conductivity of the system, when water or other solution in the sampling sub-unit is replaced by urine, wherein when said disruption takes place such event marks or triggers the initial time of the micturition event, and when such conductivity is stabilized, such stabilization event indicates that the micturition event has ended and marks the final time or termination of said event, and wherein the time interval between the initial time and the final time is used to calculate the volume of each micturition event.

10. A method for measuring the volume of one or more micturition events of an individual, wherein said method includes the steps of: measuring the conductivity of the urine passed by an individual with a non-faradaic potentiometric system comprising a pair of electrodes to which an alternate current is applied to revert the polarity of the electrodes in each half cycle; wherein the electrical conductance measurement of the urine obtained from the non-faradaic potentiometric system is then used, by previous calibration, to provide the volume of each micturition event.

11. The method of claim 10, wherein the volume of each micturition event is determined by identifying a disruption in the conductivity of the system, wherein when said disruption takes place such event marks or triggers the initial time of the micturition event, and when such conductivity is stabilized, such stabilization event indicates that the micturition event has ended and marks the final time or termination of said event, and wherein the time interval between the initial time and the final time is used to calculate the volume of each micturition event.

12. The method of any of claims 9 to 11, wherein said method includes the use of the system as defined in any of claims 1 to 12 for measuring or determining the hydration level of an individual.

**FIG. 1**

FIG 2

FIG. 3

**FIG. 4**

**FIG. 5**

Europäisches
Patentamt

European
Patent Office

Office européen
des brevets

**PARTIAL EUROPEAN SEARCH REPORT**

under Rule 62a and/or 63 of the European Patent Convention.
This report shall be considered, for the purposes of
subsequent proceedings, as the European search report

Application Number

EP 19 38 2142

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JP 2004 170331 A (TOTO LTD) 17 June 2004 (2004-06-17) * paragraphs [0014] - [0017], [0022]; figures 1-5 * ----- | 1-8 | INV. G01N33/493 G01N27/07 A61B5/20 |
| Y | US 5 038 109 A (GOBLE NIGEL M [GB] ET AL) 6 August 1991 (1991-08-06) * claims 1-4; figures 1-5 * ----- | 1-8 | |
| A | JP 2000 088845 A (TOTO LTD) 31 March 2000 (2000-03-31) * paragraphs [0021], [0022]; figure 4 * ----- | 1 | |
| A | US 5 046 510 A (AMS FELIX [DE] ET AL) 10 September 1991 (1991-09-10) * abstract; figure * ----- | 2,4 | |
| A | JP 2004 279219 A (TOTO LTD) 7 October 2004 (2004-10-07) * paragraph [0029]; figures 4, 6, 11 * ----- | 3 | TECHNICAL FIELDS SEARCHED (IPC) |
| A | JP H08 304336 A (TOTO LTD) 22 November 1996 (1996-11-22) * paragraphs [0015], [0033]; figures 3, 4, 9 * ----- | 5 | G01N A61B |

## INCOMPLETE SEARCH

The Search Division considers that the present application, or one or more of its claims, does/do
not comply with the EPC so that only a partial search (R.62a, 63) has been carried out.

Claims searched completely :

Claims searched incompletely :

Claims not searched :

Reason for the limitation of the search:

see sheet C

| Place of search | Date of completion of the search | Examiner |
|---|---|---|
| The Hague | 6 December 2019 | Wilhelm-Shalganov, J |

Europäisches
Patentamt

European
Patent Office

Office européen
des brevets

**INCOMPLETE SEARCH
SHEET C**

Application Number

EP 19 38 2142

Claim(s) completely searchable:
    1-8

Claim(s) not searched:
    9-12

Reason for the limitation of the search:

Claims 8-11 have been drafted as separate independent method claims.
Under Article 84 in combination with Rule 43(2) EPC, an application may
contain more than one independent claim in a particular category only if
the subject-matter claimed falls within one or more of the exceptional
situations set out in paragraph (a), (b) or (c) of Rule 43(2) EPC. This
is not the case in the present application.
Thus, in accordance with the applicant's request, the search has been
restricted to claims 1-8.

## ANNEX TO THE EUROPEAN SEARCH REPORT
## ON EUROPEAN PATENT APPLICATION NO.

EP 19 38 2142

This annex lists the patent family members relating to the patent documents cited in the above-mentioned European search report.
The members are as contained in the European Patent Office EDP file on
The European Patent Office is in no way liable for these particulars which are merely given for the purpose of information.

06-12-2019

| Patent document cited in search report | | Publication date | Patent family member(s) | | Publication date |
|---|---|---|---|---|---|
| JP 2004170331 | A | 17-06-2004 | NONE | | |
| US 5038109 | A | 06-08-1991 | AT | 133049 T | 15-02-1996 |
| | | | AU | 633276 B2 | 28-01-1993 |
| | | | BR | 8905181 A | 15-05-1990 |
| | | | CA | 1308782 C | 13-10-1992 |
| | | | DE | 68925454 D1 | 29-02-1996 |
| | | | DE | 68925454 T2 | 20-06-1996 |
| | | | DK | 505489 A | 14-04-1990 |
| | | | EP | 0364216 A1 | 18-04-1990 |
| | | | ES | 2082785 T3 | 01-04-1996 |
| | | | JP | H02149254 A | 07-06-1990 |
| | | | NZ | 231007 A | 23-12-1992 |
| | | | US | 5038109 A | 06-08-1991 |
| JP 2000088845 | A | 31-03-2000 | NONE | | |
| US 5046510 | A | 10-09-1991 | DE | 3926630 A1 | 21-02-1991 |
| | | | EP | 0415113 A1 | 06-03-1991 |
| | | | US | 5046510 A | 10-09-1991 |
| JP 2004279219 | A | 07-10-2004 | NONE | | |
| JP H08304336 | A | 22-11-1996 | NONE | | |

EPO FORM P0459

For more details about this annex : see Official Journal of the European Patent Office, No. 12/82